# EUROPÄISCHE PATENTSCHRIFT

(11) **EP 2 373 287 B1**
(45) Veröffentlichungstag und Bekanntmachung des Hinweises auf die Patenterteilung: **30.05.2018**
(21) Anmeldenummer: 09763855.5
(22) Anmeldetag: 24.11.2009
(51) Int. Cl.: A61K 9/00, A61K 9/16, A61K 9/14, A61K 47/14, A61K 47/44

(54) **EXTRUDATE MIT NADELFÖRMIGEN WIRKSTOFFEN**
EXTRUDATE OF NEEDLE-LIKE PHARMACEUTICAL ACTIVES
EXTRUDAT D'UN MEDICAMENT SOUS FORME D'AIGUILLE

(30) Priorität: 05.12.2008 DE 102008060472
(43) Veröffentlichungstag der Anmeldung: 12.10.2011
(73) Patentinhaber: Bayer Intellectual Property GmbH, 40789 Monheim (DE)
(72) Erfinder: KANIKANTI, Venkata-Rangarao, 51381 Leverkusen (DE); HAMANN, Hans-Juergen, 41539 Dormagen (DE); KLEINEBUDDE, Peter, 40627 Düsseldorf (DE); WITZLEB, Rieke, 14193 Berlin (DE)
(74) Vertreter: Gille Hrabal
(86) Internationale Anmeldenummer: PCT/EP2009/008341
(87) Internationale Veröffentlichungsnummer: WO 2010/063387

(56) Entgegenhaltungen:
- EP-A1- 1 690 528
- WO-A-2008/148484
- WO-A1-03/013525
- WO-A1-2005/065663
- WO-A2-2004/093801
- WO-A2-2008/063301

## Beschreibung

Die Erfindung betrifft Extrudate, enthaltend mindestens eine pharmazeutisch wirksame Substanz in Form von Nadeln, wobei das Verhältnis von Partikelgröße der nadelförmigen pharmazeutisch wirksamen Substanz zu Strangdurchmesser mindestens 1:20 beträgt, das Extrudat eine Lipidgrundlage als Hilfsstoff enthält und das Extrudat unterhalb des Schmelzbereichs der darin enthaltenen Grundlage extrudiert wurde, sowie die Verwendung dieser Extrudate zur Herstellung von Arzneimitteln.

### Stand der Technik

### Übersicht:

Adeyeye C.M., Price J.C., 1991. Development and Evaluation of Sustained-Release Ibuprofen-Wax Microspheres. 1. Effect of Formulation Variables on Physical Characteristics. Pharm. Res. 8, 1377-1383.
Adeyeye C.M., Price J.C., 1994. Development and Evaluation of Sustained-Release Ibuprofen-Wax Microspheres. 2. In-Vitro Dissolution. Pharm. Res. 11, 575-579.
Ahrens G., Mentrup E., Maas J., Radau M., 1998. Herstellung geschmackskaschierter Zubereitungen antibakteriell wirksamer Chinolonderivaten. EP 1998/0855183.
Alderborn G., Nyström C., 1982. Studies on direct compression of tablets. 3. The effect on tablet strength of changes in particle-shape and texture obtained by milling. Acta Pharm. Suec. 19, 147-156.
Al-Omran M.F., Al-Suwayeh S.A., El-Helw A.M., Saleh S.I., 2002. Taste masking of diclofenac sodium using microencapsulation. J. Microencapsul. 19, 45-52.
Barra J., Lescure F., Doelker E., 1999. Taste masking as a consequence of the organisation of powder mixes. Pharm. Acta Helv. 74, 37-42.
Barthelemy P., Laforet J.P., Farah N., Joachim J., 1999. Compritol® 888 ATO: an innovative hotmelt coating agent for prolonged-release drug formulations. Eur. J. Pharm. Biopharm. 47, 87-90.
Bienz M., 1996. Taste masking strategies for drug dosage forms. Manufacturing Chemist. 67, 17-20.
Breitkreutz J., El-Saleh F., Kiera C., Kleinebudde P., Wiedey W., 2003. Pediatric drug formulations of sodium benzoate: II. Coated granules with a lipophilic binder. Eur. J. Pharm. Biopharm. 56, 255-260.
Cabrera F.A., 2002. Solid Phase Dispersion of Quinolone-OR Naphthyridonecarboxylic acids. WO 2002/058669.
Cerea M., Zheng W.J., Young C.R., McGinity J.W., 2004. A novel powder coating process for attaining taste masking and moisture protective films applied to tablets. Int. J. Pharm. 279, 127-139.
Chun M.K., Choi H.K., 2004. Preparation and characterization of enrofloxacin/carbopol complex in aqueous solution. Arch. Pharm. Res. 27, 670-675.
Farah N., Barthelemy P., Joachim J., 1998. Method for preparing a pharmaceutical composition with modified release of the active principle, comprising a matrix. WO 1998/14176 (weiterer Stand der Technik).
Huber G.R., Jones D.R., Kuenzi J.C., Kuenzi K.D., Cabrera F.A., 2003. Animal Feeds including Actives and Methods of using same. WO 2003/030653.
Jbilou M., Ettabia A., Guyot-Hermann A.M., Guyot J.C., 1999. Ibuprofen Agglomerates Preparation by Phase Separation. Drug Dev. Ind. Pharm. 25, 297-305.
Kalbe J., Hopkins T., 1998. Oral applizierbare Granulate von Hexahydropyrazinderivaten. WO 1998/03157.
Katsuragi Y., Mitsui Y., Umeda T., Otsuji K., Yamasawa S., Kurihara K., 1997. Basic studies for the practical use of bitterness inhibitors: Selective inhibition of bitterness by phospholipids. Pharm. Res. 14, 720-724.
Kim E.-H., Choi H.K., 2004. Preparation of various solid-lipid beads for Drug Delivery of Enrofloxacin. Drug Deliv. 11, 365-370.
Li F.-Q., Hu J.-H., Deng J.-X., Su H., Xu S., Liu J.-Y., 2006. In vitro controlled release of sodium ferulate from Compritol® 888 ATO-based matrix tablets. Int. J. Pharm. 324, 152-157.
Li S.P., Martellucci S.A., Bruce R.D., Kinyon A.C., Hay M.B., Higgins J.D., 2002. Evaluation of the film-coating properties of a hydroxyethyl cellulose/hydroxypropyl methylcellulose polymer system. Drug Dev. Ind. Pharm. 28, 389-401.
Lovrecich M., Nobile F., Rubessa F., Zingone G., 1996. Effect of ageing on the release of indomethacin from solid dispersions with Eudragits. Int. J. Pharm. 131, 247-255.
Lu M.Y.F., Borodkin S., Woodward L., Li P., Diesner C., Hernandez L., Vadnere M., 1991. A Polymer Carrier System for Taste Masking of Macrolide Antibiotics. Pharm. Res. 8, 706-712.
Moisescu C., Jana C., Habelitz S., Carl G., Rüssel C., 1999. Oriented fluoroapatite glass-ceramics. J. Noncry. Sol. 248, 176-182.
Mirghani A., Idkaidek N.M., Salem M.S., Najib N.M., 2000. Formulation and release behavior of diclofenac sodium in Compritol® 888 ATO matrix beads encapsulated in alginate. Drug Dev. Ind. Pharm. 26, 791-795.
Nabil, F. et al., 1998. Method for preparing a pharmaceutical composition with modified release of the active principle, comprising a matrix method for preparing a pharmaceutical composition with modified release of the active principle, comprising a matrix. FR2753904 (A1).
Ohta M., Buckton G., 2004. The use of inverse gas chromatography to assess the acid-base contributions to surface energies of cefditoren pivoxil and methacrylate copolymers and possible links to instability. Int. J. Pharm. 272, 121-128.
Pearnchob N., Dashevsky A., Siepmann J., Bodmeier R., 2003a. Shellac used as coating material for solid pharmaceutical dosage forms: understanding the effects of formulation and processing variables. Stp Pharma Sciences. 13, 387-396.
Pearnchob N., Siepmann J., Bodmeier R., 2003b. Pharmaceutical applications of shellac: Moisture protective and taste-masking coatings and extended-release matrix tablets. Drug Dev. Ind. Pharm. 29, 925-938.
Petereit H.U., Weisbrod W., 1999. Formulation and process considerations affecting the stability of solid dosage forms formulated with methacrylate copolymers. Eur. J. Pharm. Biopharm. 47, 15-25.
Petereit H.U., Meier C., Gryczke A., 2003. Schmelzextrusion von Wirkstoffsalzen. WO 2003/072083.
Petereit H.U., Meier C., Gryczke A., 2004. Verfahren zur Herstellung einer oralen Arzneiform mit unmittelbarem Zerfall und Wirkstofffreisetzung. WO 2004/066976.
Prompruk K., Govender T., Zhang S., Xiong C.D., Stolnik S., 2005. Synthesis of a novel PEGblockpoly (aspartic acid-stat-phenylalanine) copolymer shows potential for formation of a micellar drug carrier. Int. J. Pharm. 297, 242-253.
Rasenack N., Müller B.W., 2002. Crystal habit and tableting behaviour. Int. J. Pharm. 244, 45-57.
Reitz C., Kleinebudde P., 2007. Solid lipid extrudion of sustained release dosage forms. Eur. J. Pharm. Biopharm. 67, 440-448.
Schubert M.A., Schicke B.C., Müller-Goymann C.C., 2005. Thermal analysis of the crystallization and melting behavior of lipid matrices and lipid nanoparticles containing high amounts of lecithin. Int. J. Pharm. 298, 242-254.
Shirai Y., Sogo K., Yamamoto K., Kojima K., Fujioka H., Makita H., Nakamura Y., 1993. A Novel Fine Granule System for Masking Bitter Taste. Biol. Pharm. Bull. 16, 172-177.
Sohi H., Sultana Y., Khar R.K., 2004. Taste masking technologies in oral pharmaceuticals: Recent developments and approaches. Drug Dev. Ind. Pharm. 30, 429-448.
Suzuki H., Onishi H., Takahashi Y., Iwata M., Machida Y., 2003. Development of oral acetaminophen chewable tablets with inhibited bitter taste. Int. J. Pharm. 251, 123-132.
Suzuki H., Onishi H., Hisamatsu S., Masuda K., Takahashi Y., Iwata M., Machida Y., 2004. Acetaminophen-containing chewable tablets with suppressed bitterness and improved oral feeling. Int. J. Pharm. 278, 51-61.
Takagi S., Toko K., Wada K., Ohki T., 2001. Quantification of suppression of bitterness using an electronic tongue. J. Pharm. Sci. 90, 2042-2048.
Thombre A.G., 2004. Palatable controlled-release formulations for companion animals. WO 2004/014346.
Wang H., Zhang R., 1995. Compaction behaviour of paracetamol powders of different crystal shapes. Drug Dev. Ind. Pharm. 21, 863-868.
Windbergs M., Strachan C.J., Kleinebudde P., 2008 Understanding the solid-state behaviour of triglyceride solid lipid extrudates and its influence on dissolution. Eur. J. Pharm. Biopharm. In Press.
Wong L.W., Pilpel N., 1990. The effect of particle shape on the mechanical properties of powders. Int. J. Pharm. 59, 145-154.
Yue Y., Moisescu C., Carl G., Rüssel C., 1999. Influence of suspended iso- and anisometric crystals on the flow behaviour of fluoroapatite glass melts during extrusion. Physics and Chemistry of Glasses. 40, 243-247.
Zhou F., Vervaet C., Remon J.P., 1996. Matrix pellets based on the combination of waxes, starches and maltodextrins. Int. J. Pharm. 133, 155-160.
Zhou F., Vervaet C., Schelkens M., Lefebvre R., Remon J.P., 1998. Bioavailability of ibuprofen from matrix pellets based on the combination of waxes and starch derivatives. Int. J. Pharm. 168, 79-84.

### Diskussion des Stands der Technik:

Eine Geschmacksmaskierung bitterer Arzneistoffe ist bedeutsam zur Verbesserung der Compliance bei Humanarzneimitteln, nicht zuletzt für pädiatrische Formulierungen, aber auch bei Tierarzneimitteln. Die einfachste Art der Geschmacksmaskierung ist der Zusatz von Aromen, was bei sehr bitteren und sehr gut wasserlöslichen Stoffen problematisch sein kann (Bienz, 1996). Auch die Geschmacksmaskierung durch Verarbeitung eines Wirkstoffs mit einem hydrophoben Träger zu Granulaten wurde beschrieben (WO 1998/03157 Kalbe und Hopkins, 1998). Eine andere Möglichkeit ist das Überziehen von Arzneiformen. Dazu verwendete Materialien sind zum Beispiel Eudragit E (Cerea et al., 2004; Lovrecich et al., 1996; Ohta und Buckton, 2004; Petereit und Weisbrod, 1999), Schellack (Pearnchob et al., 2003b; Pearnchob et al., 2003a) oder Cellulosederivate (Al-Omran et al., 2002; Li et al., 2002; Shirai et al., 1993). Der Nachteil von Eudragit E ist, dass die Geschmacksmaskierung auf einer ionischen Wechselwirkung zwischen kationischem Hilfsstoff und anionischen Wirkstoffen beruht. Der Nachteil von Schellack ist, dass es sich um ein natürliches Polymer handelt, dessen Zusammensetzung variieren kann. Abgesehen davon stellt das Überziehen von Arzneiformen einen zusätzlichen kosten- und zeitintensiven Arbeitsschritt dar. Außerdem wurden feste Dispersionen von Chinolon- oder Naphthyridoncarbonsäuren in einer unlöslichen Schellack-Matrix beschrieben (WO 2002/058669 Cabrera, 2002).

Ionenaustauscherharze und Einschlusskomplexe werden ebenfalls für eine Geschmacksmaskierung verwendet. Die Anwendbarkeit der Ionenaustauscherharze ist dadurch eingeschränkt, dass ionische Eigenschaften des Arzneistoffs vorliegen müssen (Chun und Choi, 2004; Lu et al., 1991; Prompruk et al., 2005). Einschlusskomplexe können nur mit geringen Mengen an Arzneistoffen beladen werden (Sohi et al., 2004).

Lipidgrundlagen können ebenfalls zur Geschmacksmaskierung eingesetzt werden. Beschrieben sind monolithische Arzneiformen auf Grundlage von Hartfett, die zusätzlich Lecithin und Süßstoffe zur Geschmacksmaskierung enthalten (Suzuki et al., 2003; Suzuki et al., 2004). Der Nachteil dabei ist, dass die Lipide während der Herstellung vollständig geschmolzen werden müssen, was zu physischen Instabilitäten führen kann. Des Weiteren wurden Hartfett, Glyceroldistearat und Stearinsäure als lipophile Bindemittel in der Kalt-Extrusion verwendet, wobei auch hier ein Einsatz von Eudragit E als Überzug nötig war, um eine Geschmacksmaskierung zu erreichen (Breitkreutz et al., 2003). Die Extrusion von Fetten unterhalb ihres Schmelzpunktes zur Herstellung von Arzneiformen wurde ebenfalls beschrieben, allerdings nicht zum Zwecke einer Geschmacksmaskierung (Reitz und Kleinebudde, 2007; Windbergs et al., 2008).

Geschmacksmaskierte Formulierungen mit Gyraseinhibitoren vom Chinolon-Typ wurden erhalten, indem der Wirkstoff mit höheren Fettsäuren und gegebenenfalls weiteren Additiven gemischt, erhitzt und nach Abkühlen granuliert oder pulverisiert wurde (EP 1998/0855183 Ahrens et al., 1998). Weiterhin wurden Pellets auf der Grundlage von Wachsen hergestellt (Adeyeye und Price, 1991; Adeyeye und Price, 1994; Zhou et al., 1996; Zhou et al., 1998). Die Untersuchungen ergaben, dass die Freisetzung der Wirkstoffe von dem Schmelzpunkt des verwendeten Wachses und dessen Konzentration im Pellet abhängt. Je höher der Schmelzpunkt und der Gehalt an Wachs waren, desto langsamer war die Freisetzung. Eine weitere Möglichkeit der Geschmacksmaskierung beschreiben Kim und Choi (2004), die einen Kern aus Kakaobutter oder Hartfett und dem Wirkstoff herstellten und diesen mit einem Überzug aus Natriumalginat oder Carrageenan versahen. Dabei wurde jedoch das Fett vollständig geschmolzen, was aus Stabilitätsgründen ungünstig war, außerdem stellte der Herstellungsschritt des Überziehens einen zusätzlichen Arbeitsschritt dar.

Weiterhin wurde Compritol® 888 ATO als matrixbildende Komponente beschrieben. Die Pellets bestanden aus geschmolzenem Compritol®, dem Wirkstoff und einem Polysaccharidüberzug (Mirghani et al., 2000). In einer anderen Studie wurden Matrixtabletten entweder direkt aus einer Pulvermischung oder einer pulverisierten festen Dispersion verpresst. Die Tabletten aus der pulverisierten festen Dispersion zeigten eine bessere Geschmacksmaskierung, für deren Herstellung mußte das Compritol® allerdings vollständig geschmolzen werden (Li et al., 2006). Barthelemy verwendete Compritol® zum Überziehen von Theophyllinpellets und -granulaten. Auch hier wurde das Fett vollständig geschmolzen (Barthelemy et al., 1999).

Der Einsatz von Phospholipiden stellt eine weitere Möglichkeit der Geschmacksmaskierung von bitterem Geschmack dar, nicht dagegen von anderen Geschmacksrichtungen (Katsuragi et al., 1997; Takagi et al., 2001). Außerdem beeinflusst der Zusatz von Phospholipiden die Kristallinität der Lipide, was zu Instabilitäten führen kann (Schubert, 2005). Eine Geschmacksmaskierung von Pulvern ist möglich, indem kleine Hilfsstoffpartikel auf größere Wirkstoffpartikel aufgelagert werden (Barra et al., 1999).

Beschrieben wurde weiterhin Tierfutter, in welches Wirkstoffe mittels Extrusion eingearbeitet wurden (WO 2003/030653 Huber et al., 2003). Petereit et al. erzielten mittels Schmelzextrusion eines basischen Arzneistoffs und eines Methacrylatpolymers geschmacksmaskierte Extrudate, die anschließend zu einem Granulat oder Pulver zerkleinert wurden (WO 2003/072083 Petereit et al., 2003) und eine schnell zerfallende Arzneiform durch Mischen der beiden Komponeneten mit einer mittel- bis langkettigen Fettsäure in der Schmelze. Nach dem Erstarren wurde das Produkt gemahlen und in eine wasserlösliche Matrix eingebettet (WO 2004/066976 Petereit et al., 2004). Untersucht wurden Arzneimittel mit kontrollierter Freisetzung, die den Wirkstoff in einer Lipidmatrix aus Behensäureester und einem hydrophoben Verdünnungsmittel enthielten (FR2753904 (A1) Nabil et al., 1998). Thombre beschreibt eine Formulierung für Hobbytiere in multipartikulärer Form mit einem geschmacksmaskierenden Zusatz (WO 2004/014346 Thombre, 2004).

In der anhängigen Anmeldung "Extrudate mit verbesserter Geschmacksmaskierung" (Deutsche Patentanmeldung Akz. 102007026550.8, siehe auch korrespondierende PCT-Anmeldung Nr. PCT/EP2008/004218) wurden pharmazeutisch verwendbare Extrudate mit einem Strangdurchmesser von 0,5 mm und weniger beschrieben. Diese Extrudate eignen sich zur Geschmacksmaskierung von Arzneistoffen.

Die EP 1 690 528 A1 betrifft Verfahren zur Herstellung von Dosierungsformen, die eine feste Dispersion eines mikrokristallinen Wirkstoffs umfasst, bei dem ein thermoplastisches Polymer mit einer Glasübergangstemperatur (Tg) von wenigstens 40°C geschmolzen und ein Wirkstoff in der Schmelze homogen gelöst wird, in der erhaltenen Masse Kristallisation des Wirkstoffs bei einer Temperatur bei oder oberhalb der Tg des Polymers initiiert und die Masse auf eine Temperatur unterhalb der Tg des Polymers abkühlt.

Bei der Extrusion von Gemischen, die Wirkstoffe in Nadelform enthalten, ergeben sich jedoch unerwartete Schwierigkeiten: Ist die Kristallform der eingesetzten Arzneistoffe nadelförmig, lassen sich keine stabilen und reproduzierbaren Herstellungsprozesse durchführen, auch wenn die Länge der Nadeln deutlich kleiner ist als der Strangdurchmesser. Grundsätzlich waren Verarbeitungsschwierigkeiten mit nadelförmigen Arzneistoffen bei anderen Herstellungstechnologien bekannt. In verschiedenen Arbeiten ist gezeigt worden, dass die Eigenschaften von Pulvern und resultierenden Tabletten von der Partikelform der eingesetzten Stoffe abhängen (Alderborn und Nyström, 1982; Wong und Pilpel, 1990). Paracetamol zum Beispiel ließ sich als nadelförmiger Habitus sehr viel schlechter zu Tabletten verpressen als in anderen Kristallformen, was sich in deckelnden Tabletten und schlechter Fließfähigkeit des Pulvers äußerte (Wang & Zhang, 1995). Ibuprofen zeigte in nadelförmiger Kristallform ebenfalls eine sehr schlechte Fließfähigkeit, kohäsive und adhäsive Eigenschaften, zum Kompaktieren und Tablettieren war ein hoher Energieeintrag nötig und die resultierenden Tabletten waren mechanisch instabil. Durch Agglomeration oder Umkristallisation in isometrische Kristallformen ließen sich die Tablettiereigenschaften von Ibuprofen deutlich verbessern (Jbilou et al., 1999; Rasenack und Müller, 2002).

Bezüglich des Extrusionsprozesses war aber aus der Glasverarbeitung bekannt, dass nadelförmige Partikel sich in Extrusionsrichtung anordnen. Eine Ausrichtung war in diesem Fall erwünscht, um anisotrope Eigenschaften der hergestellten Gläser zu erzielen (Moisescu et al., 1999). Im Vergleich mit isometrischen Partikeln war die Viskosität der erweichten Glasmasse während der Schmelzextrusion deutlich höher, wenn nadelförmige Partikel enthalten waren (Yue et al., 1999).

Der Fachmann hätte keine Probleme erwartet, sofern die Teilchengröße um einen deutlichen Faktor (z. B. 5) kleiner ist als der Strangdurchmesser, insbesondere, da er ja annehmen konnte, dass sich die Teilchen für die Extrusion günstig parallel ausrichten würden.

Überraschenderweise ergeben sich aber bei der Extrusion von Mischungen mit nadelförmigen Wirkstoffen Probleme: Die Probleme im Herstellungsprozess äußern sich in Form von Stauungen vor der Düsenplatte, sodass Düsenlöcher blockiert werden und dadurch bedingt der Druck vor der Düsenplatte des Extruders steigt. Die Pulvermischungen sind außerdem so schlecht fließend, dass eine konstante Dosierrate bei hohen Prozessgeschwindigkeiten kaum aufrecht zu erhalten ist.

Um zu einem befriedigenden Extrusionsverfahren für nadelförmige Wirkstoffe zu kommen, sind verschiedene Lösungsansätze möglich: Änderungen an der Rezeptur, z. B. Variation der Lipidgrundlage, Zusatz weiterer Hilfsstoffe oder Versuche mit verschiedenen Wirkstoff-Beladungen bringen keine ausreichenden Verbesserungen. Des Weiteren können Düsenplatten mit einer stufenweisen Aufweitung des Düsenkanals in Prozessrichtung verwendet werden, was allerdings im vorliegenden Fall unter Umständen zu einer Verschlechterung der Prozessgleichmäßigkeit führt. Auch Extrusion durch Düsenplatten mit besonders glatten Oberflächen der Lochbohrungen hat keine wesentliche Änderung zur Folge. Als weitere apparative Variation können verschiedene Schneckenkonfigurationen verwendet werden, was ebenfalls zu keiner Verbesserung führt. Ferner können die Prozessparameter Temperatur, Dosierrate und Schneckendrehzahl variiert werden. Extrusionstemperaturen von 20°C unterhalb des Schmelzbereichs des eingesetzten Lipids bis in den Schmelzbereich hinein sind möglich. Bei zu niedriger Temperatur verstopfen die Düsen sofort und der Druck steigt sehr schnell an, bei zu hoher Temperatur schmelzen die Lipide vollständig und verlassen die Düsen als weiche Paste.

Allerdings zeigt sich, dass mit größeren Düsendurchmessern gewisse Verbesserungen erzielt werden. Da keine der anderen Veränderungen den Prozess entscheidend verbessert, wird das Wirkstoffpulver gemahlen (z. B. in einer Luftstrahlmühle); durch die Mahlung werden die nadelförmigen Kristallstrukturen zerkleinert. Es zeigt sich, dass bei genügend kleiner Partikelgröße die Extrudate problemlos hergestellt werden können. Der Extrusionsprozess mit ausreichend fein gemahlenem Wirkstoff verläuft in der Regel gleichmäßig und reproduzierbar mit konstantem Druck, sogar bei hoher Arzneistoffbeladung (z. B. 50 % oder sogar bis zu 80 %) und einem Düsendurchmesser von z. B. 0,3 mm oder sogar nur 0,2 mm.

Die Erfindung betrifft daher:
- Extrudate, enthaltend mindestens eine pharmazeutisch wirksame Substanz in Form von Nadeln, dadurch gekennzeichnet, dass das Verhältnis von Partikelgröße der nadelförmigen pharmazeutisch wirksamen Substanz zu Strangdurchmesser mindestens 1:20 beträgt,
   dass das Extrudat eine Lipidgrundlage als Hilfsstoff enthält, und
   dass das Extrudat unterhalb des Schmelzbereichs der darin enthaltenen Grundlage extrudiert wurde.
- Die Verwendung der vorstehend genannten Extrudate zur Herstellung von Arzneimitteln.

Das Verhältnis von Partikelgröße der nadelförmigen pharmazeutisch wirksamen Substanzen zum Strangdurchmesser beträgt üblicherweise mindestens 1:15, gemäß der vorliegenden Erfindung wie beansprucht mindestens 1:20, besonders bevorzugt mindestens 1:25, ganz besonders bevorzugt mindestens 1:50, insbesondere mindestens 1:100.

Unter Partikelgröße soll hier im Zweifel der durch Laserdiffraktometrie bestimmte d(0,9)-Wert verstanden werden. Im Sinne dieser Erfindung wird unter d(0,9) eine volumenbezogene Partikelgrößenverteilung verstanden, bei der 90 % aller Partikel eine Dimension (Durchmesser) kleiner oder gleich diesem Wert aufweisen (Gelegentlich werden dafür auch die Angaben d(90) oder d(v,90) verwendet, letzteres um deutlich zu machen, dass es sich um eine volumenbezogene Partikelgrößenverteilung handelt). Entsprechend sind die Bezeichnungen d(0,5), d(0,1) usw. zu verstehen. Die hier angegebenen Partikelgrößen wurden mit der Methode der Laserbeugung mit dem Gerät Mastersizer 2000 (Dispergiereinheit Hydro 2000G) der Firma Malvern und dem Auswertemodus der Fraunhoferbeugung bestimmt, da die Brechungsindices der Wirkstoffpartikel nicht bekannt sind. Eine geeignete Menge der Probenlösung wurde dabei mit 2-3 ml eines Dispergiermediums (z. B. eine 0,1 % wässrige Dioctylnatriumsulfosuccinat-Lösung für Praziquantel oder Ethanol für Mesalazin) unter Rühren vordispergiert. Die Dispersion wurde dann unter Rühren (300 UpM) und Umpumpen (900 UpM) in die Dispergiereinheit des Geräts gegeben und vermessen. Die Auswertesoftware gab die Partikelgröße als d(0,9)-Werte (bzw. d(0,5)-Werte usw.) aus.

Wirkstoffpartikel, die in gängigen Lösungsmitteln zu gut löslich sind. (z. B. Coffein), werden mit einer geeigneten Einheit (z. B. Scirocco 2000 dry powder feeder) mittels eines Luftstroms bei 0,5 bar Luftdruck trocken dispergiert.

Der Strangdurchmesser der erfindungsgemäßen Extrudate beträgt vorzugsweise höchstens 0,5 mm, besonders bevorzugt höchstens 0,3 mm. Üblicherweise können Extrudate ab einem Durchmesser von 0,1 mm, vorzugsweise ab 0,2 mm verwendet werden. Bei nichtzylindrischen Extrudaten beträgt die maximale Kantenlänge oder Ellipsenlänge höchstens 0,5 mm, bevorzugt höchstens 0,3 mm.

Die Extrudate enthalten eine für die Extrusion geeignete Grundlage aus einem thermoplastisch verformbaren Material oder einer Mischung mehrerer thermoplastisch verformbarer Materialien sowie gegebenenfalls weitere pharmazeutisch annehmbare Hilfs- und Zusatzstoffe.

Die Grundlage besteht aus thermoplastisch verformbaren Materialien wie Polymeren, beispielsweise Polyacrylaten oder Cellulosederivaten, Lipiden, beispielsweise Acylglyceriden, Tensiden, beispielsweise Glycerolmonostearat oder Natriumstearat, Macrogolen, beispielsweise Polyethylenglykol 6000, Zuckern bzw. Zuckeralkoholen, beispielsweise Mannitol oder Xylitol. Erfindungsgemäß wie in den Ansprüchen beansprucht enthält das Extrudat eine Lipidgrundlage. Als Lipidgrundlage eignen sich z. B. Fettgrundlagen, insbesondere Glycerolester, bevorzugt handelt es sich um Ester mit C₁₂-C₂₄-Fettsäuren. Als Glycerolester seien genannt Glyceroldiester, wie z. B. Glyceroldibehenat; Glyceroltriester, wie z. B. Glyceroltrilaurat, Glyceroltrimyristat, Glyceroltripalmitat oder Glyceroltristearat, Mischungen aus Glycerolmono-, -di- und -triestern, wie z. B. Glycerolpalmitostearat. Auch genannt seien Triglyceride auf Basis von Kokosfett, Palmöl und/oder Palmkernöl (wie z. B. die unter der Bezeichnung Witocan® im Handel befindlichen Hartfette). Auch Mono- oder Diglyceride von Zitronen- und/oder Milchsäure sind einsetzbar.

Weiterhin seien genannt Wachse, insbesondere solche mit 30 bis 60 Kohlenstoffatomen, wie Cetylpalmitat.

Solche Lipide sind z. B. kommerziell unter den Bezeichnungen Precirol®, Compritol® und Dynasan® erhältlich.

Ein besonders bevorzugtes Beispiel aus der Reihe der Glyceroldiester ist Glyceroldibehenat (z. B. Compritol® 888 ATO, das hauptsächlich Glyceroldibehenat aber auch Glycerolmonobehenat und Glyceroltribehenat enthält). Besonders bevorzugte Beispiele aus der Reihe der Glyceroltriester sind Glyceroltrimyristat (z. B. Dynasan® 114), Glyceroltripalmitat (z. B. Dynasan® 116) und Glyceroltristearat (z. B. Dynasan® 118).

Bevorzugt sind die Fettgrundlagen pulverförmig. Viele Lipide sind polymorph und können bei Temperatur- und Druckänderungen unter Umständen metastabile Formen bilden. Bei Lagerung können unter Umständen Umwandlungen der Modifikationen auftreten und sich stabilere Modifikationen bilden. Laut Beschreibungen in der Literatur [Reitz und Kleinebudde, 2007; Windbergs et al., 2008] sind Glyceroltriester (z. B. bekannt als Dynasan®), insbesondere Glyceroltrimyristat (Dynasan 114®) oder auch Glyceroltripalmitat (z. B. Dynasan® 116) oder Glyceroltristearat (z. B. Dynasan® 118), vergleichsweise stabil gegen solche Änderungen und eignen sich daher besonders als Lipidgrundlage für Arzneimittel.

Insbesondere die als Fettgrundlagen verwendeten Stoffe kommen oft als Mischungen z. B. von Mono-, Di- und/oder Triglyceriden in den Handel. Gegenüber diesen sind einheitliche Fettgrundlagen, die im wesentlichen nur aus einer Komponente bestehen, bevorzugt. Mit diesen Hilfsstoffen hergestellte Formulierungen zeichnen sich durch eine gute Lagerstabilität aus.

Die eingesetzte Menge an Grundlage (aus thermoplastisch verformbaren Materialien) hängt von der Menge der sonstigen Inhaltsstoffe der Extrudate ab. Üblicherweise werden 15 bis 99 % [m/m], bevorzugt 20 bis 99 % [m/m], besonders bevorzugt 25 bis 80 % [m/m], ganz besonders bevorzugt 30 bis 70 % [m/m] eingesetzt.

Die eingesetzten Grundlagen, insbesondere die Lipidgrundlagen, haben in der Regel einen Schmelzbereich, dessen Untergrenze üblicherweise bei mindestens 50°C, bevorzugt bei mindestens 60°C liegt.

Die erfindungsgemäßen Extrudate können gegebenenfalls einen oder mehrere weitere Hilfs- und Zusatzstoffe enthalten. Als solche kommen in Frage: Fließregulierungsmittel, bevorzugt kolloidales Siliciumdioxid in einer Konzentration von 0,2 bis 2 % [m/m]; Schmiermittel, bevorzugt Magnesiumstearat oder Calciumdibehenat in einer Konzentration von 0,2 bis 5 % [m/m]; Tenside, bevorzugt Lecithin in einer Konzentration von 0,5 bis 10 % [m/m]. Weiterhin können Antioxidantien eingesetzt werden, es eignen sich beispielsweise Butylhydroxyanisol (BHA) oder Butylhydroxytoluol (BHT), die in üblichen Mengen verwendet werden, in der Regel 0,01 bis 0,5 % [m/m], bevorzugt 0,05 bis 0,2 % [m/m]. Die Wirkstofffreisetzung lässt sich beispielsweise durch Zusatz von sogenannten Porenbildnern steuern. Dies sind z. B. Zucker, besonders Lactose, Polyole, insbesondere Mannit oder Polyethylenglykole (PEG), bevorzugt PEG 1500 bis 10000, besonders bevorzugt PEG 1500 bis 6000, z. B. PEG 1500 (Macrogol 1500). Die Porenbildner werden in einer Konzentration von 5 bis 40 % [m/m], bevorzugt in einer Konzentration von 5 bis 20 % [m/m] eingesetzt. Eine andere Möglichkeit der Beeinflussung der Wirkstofffreisetzung stellt der Zusatz von Zerfallshilfsmitteln dar. Dazu können sogenannte Supersprengmittel wie Crospovidon, Croscarmellose-Natrium oder quervernetzte Natriumcarboxymethylstärke eingesetzt werden. Die Supersprengmittel werden in einer Konzentration von 1 bis 15 % [m/m], bevorzugt in einer Konzentration von 3 bis 10 % [m/m] eingesetzt. Alternativ dazu können Stoffe eingesetzt werden, die im Sauren löslich sind und/oder Kohlendioxid entwickeln wie Magnesiumcarbonat oder Calciumcarbonat. Die Kohlendioxid freisetzenden Stoffe werden in einer Konzentration von 5 bis 15 % [m/m], bevorzugt in einer Konzentration von 5 bis 10 % [m/m] eingesetzt.

Weiterhin können die erfindungsgemäßen Extrudate Antistatika enthalten. Dies ist insbesondere dann empfehlenswert, wenn elektrostatische Aufladungen die Extrusion beeinträchtigen. Elektrostatische Aufladungen können zum Verstopfen der Düsenlöcher führen, was sich durch Zusatz von Antistatika verhindern lässt. Als Antistatika können vorzugsweise PEG zugesetzt werden, insbesondere kommen in Frage PEG 1500 bis 6000. Das PEG sollte bevorzugt pulverförmig vorliegen und während der Extrusion schmelzen, um einen antistatischen Effekt zu entfalten. Bei Zusatz von PEG als Antistatikum sollte daher die Schmelztemperatur des PEG niedrig genug sein, dass bei der Extrusion zwar das PEG aber nicht die verwendete Fettgrundlage schmilzt. In der Praxis treten statische Aufladungen nicht bei allen Grundlagen auf. Sie werden vor allem bei Glycerol-Fettsäure-Triestern mit Fettsäuren, wie z. B. Glyceroltrimyristat, Glyceroltripalmitat oder Glyceroltristearat, beobachtet.

Bei Verwendung solcher Grundlagen empfiehlt es sich daher, der zu extrudierenden Mischung ein Antistatikum beizugeben, um eine problemlose Extrusion zu gewährleisten. Die Antistatika werden in einer Konzentration von mindestens 5 % [m/m], bevorzugt von mindestens 10 % [m/m] eingesetzt. Üblicherweise werden nicht mehr als 30 % [m/m] eingesetzt.

Als pharmazeutisch wirksame Substanzen können Arzneimittelwirkstoffe eingesetzt werden und zwar insbesondere solche, deren unangenehmer Geschmack kaschiert werden soll.

Erfindungsgemäß werden Wirkstoffe eingesetzt, die in Nadelform vorliegen. In aller Regel sind das nadelförmige Kristalle.

Unter "Nadel" bzw. "nadelförmig" sollen hier Teilchen verstanden werden, deren Länge deutlich größer ist als ihr Durchmesser, wobei das Längen/Durchmesser-Verhältnis mindestens größer als 3:1, bevorzugt größer als 5:1, besonders bevorzugt größer als 10:1, ganz besonders bevorzugt größer als 20:1 ist. Da die Nadeln in der Regel nicht rund sind, soll unter "Durchmesser" im Zweifel die größte Abmessung senkrecht zur Länge verstanden werden.

Grundsätzlich ist man in der Wahl des nadelförmigen Wirkstoffs nicht sehr eingeschränkt, da es nicht erforderlich ist, den Wirkstoff zu schmelzen. Aufgrund der geschmacksmaskierenden Wirkung der Extrudate eignen sie sich bevorzugt für unangenehm - z. B. bitter - schmeckende Wirkstoffe.

Die Wirkstoffe können z. B. zu folgenden Gruppen gehören: Antibiotika, Mittel gegen parasitische Protozoen, Anthelmintika, Stoffwechsel-stimulierende Mittel, entzündungshemmende Stoffe.

Selbstverständlich schließt der Begriff Wirkstoff auch nadelförmige Salze und Solvate ein.

Als konkretes Beispiel für einen Wirkstoff sei Mesalazin genannt. Mesalazin ist ein entzündungshemmender Arzneistoff, der gegen chronisch-entzündliche Darmerkrankungen eingesetzt wird. Es ist sehr schwer löslich in Wasser, hat einen Schmelzpunkt von 280°C und eine nadelförmige Kristallform.

Ein weiteres Beispiel ist kristallines Coffein.

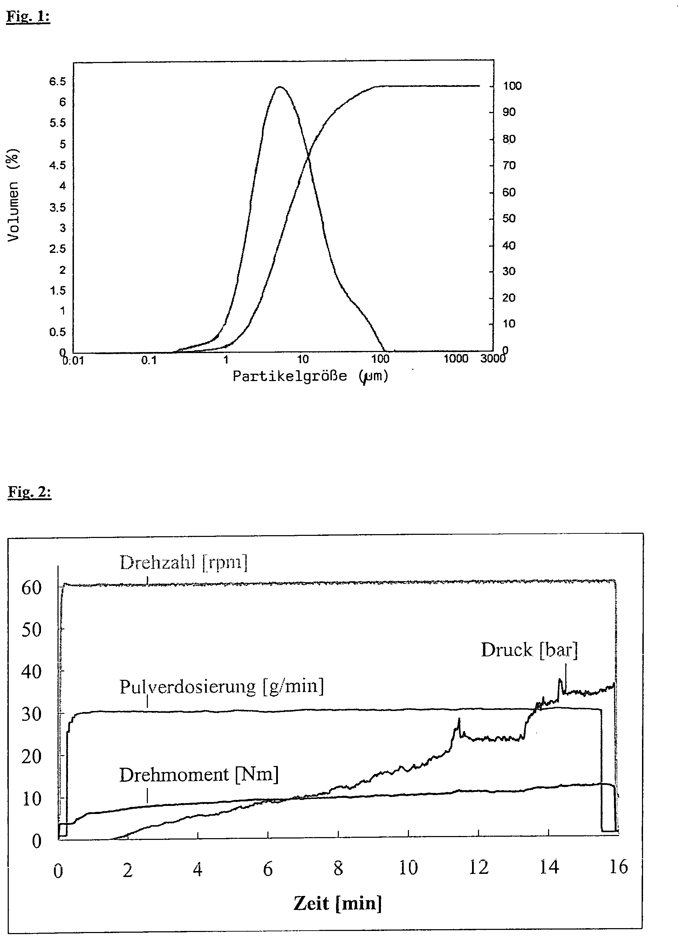

Als bevorzugtes konkretes Beispiel für einen nadelförmigen Wirkstoff sei Praziquantel genannt, ein lange bekanntes Anthelmintikum, das gegen Bandwürmer und Schistosomen wirkt. Es ist schwer löslich in Wasser, hat einen Schmelzpunkt von 139°C und eine nadelförmige Kristallform. Üblicherweise eingesetzt wird eine in einer Stiftmühle gemahlene Qualität mit einer Partikelgröße von 25 µm, ausgedrückt als d(0,9), gemessen per Laserdiffraktometrie (Fig. 1).

Durch die Einbettung in eine lipophile Matrix kann - je nach Natur des eingesetzten Wirkstoffs - auch eine verzögerte Freisetzung und damit ein Retard-Effekt erreicht werden.

Die in den Extrudaten eingesetzte Menge an Wirkstoff hängt von der Wirkstärke und der angestrebten Dosierung ab. Es zeigt sich, dass auch Extrudate mit hohen Wirkstoffkonzentrationen von bis zu 80 % [m/m], bevorzugt bis zu 70 % [m/m], besonders bevorzugt bis zu 60 % [m/m] hergestellt werden können. Übliche Konzentrationsbereiche sind z. B. 1 bis 80 % [m/m], bevorzugt 5 bis 70 % [m/m], besonders bevorzugt 30 bis 60 % [m/m].

Die erfindungsgemäßen Extrudate werden hergestellt, indem man die Ausgangsmaterialien (die pharmazeutisch wirksame(n) Substanz(en), die Grundlage und ggf. Hilfs- und Zusatzstoffe) mischt und dann extrudiert. Die Extrusionen werden bevorzugt bei einer Temperatur durchgeführt, die nicht zu einem vollständigen Schmelzen der thermoplastisch verformbaren Materialien führt und zwar üblicherweise bei einer Temperatur im Bereich von Raumtemperatur, bevorzugt von 40°C, bis unterhalb des Schmelzbereichs der thermoplastisch verformbaren Materialien. In der Praxis bezieht man sich dabei üblicherweise auf den für die jeweilige Grundlage angegebenen Schmelzbereich. In der Regel wird die Extrusionstemperatur nicht niedriger als 20° C, bevorzugt 15° C, besonders bevorzugt 10° C unterhalb der Untergrenze des Schmelzbereichs der Grundlage, insbesondere der Fettgrundlage, eingestellt. Erfindungsgemäß wird die Extrusionstemperatur nicht höher als die Untergrenze des Schmelzbereichs der Grundlage, bevorzugt 1° C tiefer, besonders bevorzugt 5°C tiefer eingestellt. Das Ziel ist die Extrusion einer weichen Paste zu vermeiden. Der Extrusionsprozess sollte bei möglichst konstanter Materialtemperatur durchgeführt werden. Für diesen Zweck eignen sich besonders beheizbare Schneckenextruder, insbesondere Zweischneckenextruder. Der extrudierte Strang hat bevorzugt einen runden Querschnitt und einen Durchmesser wie oben angegeben. Der extrudierte Strang kann direkt bei der Extrusion mit einem Messer oder in einem separaten Schritt durch schonendes Mahlen in einer üblichen Mühle, z. B. in einer Zentrifugalmühle, zerkleinert werden. Die Korngröße des erhaltenen Produkts hängt vom Durchmesser der verwendeten Düse ab, wobei die zerkleinerten Stränge maximal eine Länge aufweisen, die dem Dreifachen des Strangdurchmessers entspricht. Typische Korngrößen sind z. B. 300 bis 500 µm oder bei kleinerem Düsendurchmesser auch 200 bis 500 µm. Gemäß einer bevorzugten Ausführungsform kann das Mahlgut noch gesiebt werden. Dadurch kann der Feinanteil entfernt werden.

Die hierin verwendete Angabe, dass die Extrudate unterhalb ihres Schmelzpunktes extrudiert werden, ist so zu verstehen, dass die Extrudate - wie oben angegeben - bei einer Temperatur extrudiert werden, bei der die eingesetzte thermoplastische Grundlage noch nicht vollständig schmilzt. Oft haben andere Bestandteile, wie z. B. die Wirkstoffe, einen höheren Schmelzpunkt. Solche Extrudate eignen sich zur Geschmackskaschierung unangenehm schmeckender Inhaltsstoffe.

Die erfindungsgemäßen Extrudate können nach schonender Zerkleinerung gegebenenfalls zu geeigneten Arzneimittelformen weiter verarbeitet werden. Für die weitere Verarbeitung ist gegebenenfalls der Zusatz weiterer Hilfsstoffe erforderlich. Die erfindungsgemäß bevorzugte Arzneimittelform sind Tabletten, die ggf. an die gewünschte Anwendung angepasste Formen haben können. Andere Arzneimittelformen, die in Frage kommen sind Pasten, Suspensionen, Sachets, Kapseln etc..

Die erfindungsgemäßen Extrudate bzw. Arzneimittel eignen sich generell für die Anwendung bei Mensch und Tier. Bevorzugt werden sie in der Tierhaltung und Tierzucht bei Nutz-, Zucht-, Zoo-, Labor-, Versuchs- und Hobbytieren eingesetzt, und zwar insbesondere bei Säugetieren.

Zu den Nutz- und Zuchttieren gehören Säugetiere wie z. B. Rinder, Pferde, Schafe, Schweine, Ziegen, Kamele, Wasserbüffel, Esel, Kaninchen, Damwild, Rentiere, Pelztiere wie z. B. Nerze, Chinchilla, Waschbär, sowie Vögel wie z. B. Hühner, Gänse, Puten, Enten, Tauben und Straußenvögel. Beispiele für bevorzugte Nutztiere sind Rind, Schaf, Schwein und Huhn.

Zu Labor- und Versuchstieren gehören Hunde, Katzen, Kaninchen und Nagetiere wie Mäuse, Ratten, Meerschweinchen und Goldhamster.

Zu den Hobbytieren gehören Hunde, Katzen, Pferde, Kaninchen, Nagetiere wie Goldhamster, Meerschweinchen, Mäuse, des Weiteren Reptilien, Amphibien und Vögel zur Heim- und Zoohaltung.

Die Anwendung der Extrudate erfolgt üblicherweise direkt oder in Form von geeigneten Zubereitungen (Arzneimittelformen) enteral, insbesondere oral.

Die enterale Anwendung geschieht z. B. oral in Form von Granulaten, Tabletten, Kapseln, Pasten, Granulaten, Suspensionen oder medikiertem Futter. Eine Möglichkeit der oralen Applikation ist das sogenannte Topdressing, dabei handelt es sich um ein Pulver, Granulat oder um eine Paste, die auf das Futter gegeben und mit dem Futter aufgenommen wird.

Geeignete Zubereitungen sind:
feste Zubereitungen wie zum Beispiel Granulate, Pellets, Tabletten, Boli und wirkstoffhaltige Formkörper.

Zur Herstellung fester Zubereitungen werden die zerkleinerten Extrudate mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z. B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z. B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe. Geeignete Hilfsstoffe und die erforderlichen Einsatzmengen sind dem Fachmann grundsätzlich bekannt. Als Konservierungsstoff sei z. B. Sorbinsäure genannt. Als Antioxidantien eignen sich beispielsweise Butylhydroxyanisol (BHA) oder Butylhydroxytoluol (BHT). Als Farbstoffe kommen für pharmazeutische Zwecke geeignete organische und anorganische Farbstoffe bzw. Pigmente in Frage, wie z. B. Eisenoxid.

Weitere geeignete Hilfsstoffe sind Schmier- und Gleitmittel wie z. B. Magnesiumstearat, Stearinsäure, Talkum, Bentonite, zerfallsfördernde Substanzen wie Stärke oder quervernetztes Polyvinylpyrrolidon, Bindemittel wie z. B. Stärke, Gelatine oder lineares Polyvinylpyrrolidon sowie Trockenbindemittel wie mikrokristalline Cellulose.

Als weitere Hilfsmittel können Öle wie Pflanzenöle (z. B. Olivenöl, Sojaöl, Sonnenblumenöl) oder Öle tierischer Herkunft wie z. B. Fischöl eingesetzt werden. Übliche Mengen sind 0,5 bis 20 % [m/m], bevorzugt 0,5 bis 10 % [m/m], besonders bevorzugt 1 bis 2 % [m/m].

Suspensionen können oral angewendet werden. Sie werden hergestellt, indem man die zerkleinerten Extrudate in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten kommen homogene Lösungsmittel oder Lösungsmittelgemische in Frage, in denen sich die jeweiligen Extrudate nicht lösen. Beispielhaft seien genannt physiologisch verträgliche Lösungsmittel wie Wasser, Alkohole wie Ethanol, Butanol, Glycerin, Propylenglykol, Polyethylenglykole sowie Gemische derselben.

Als Netzmittel (Dispergiermittel) können Tenside eingesetzt werden. Beispielhaft seien genannt:
nichtionogene Tenside, z. B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie z. B. Di-Na-N-lauryl-β-iminodipropionat oder Lecithin;
anionaktive Tenside, wie z. B. Na-Laurylsulfat, Fettalkoholethersulfate, Mono/Dialkylpolyglykoletherorthophosphorsäureester - monoethanolaminsalz;
kationaktive Tenside wie z. B. Cetyltrimethylammoniumchlorid.

Als weitere Hilfsstoffe seien z. B. genannt:
Viskositätserhöhende und die Suspension stabilisierende Stoffe wie Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Halbfeste Zubereitungen können oral verabreicht werden. Sie unterscheiden sich von den oben beschriebenen Suspensionen und Emulsionen nur durch ihre höhere Viskosität.

Die Wirkstoffe können auch in Kombination mit Synergisten oder mit weiteren Wirkstoffen eingesetzt werden.

### Beispiele

Soweit nicht anders angegeben handelt es sich bei den Prozentangaben um Gewichtsprozent bezogen auf die fertige Mischung.

### I. Vergleichsbeispiel: Extrudat mit Praziquantel:

Eine Pulvermischung bestehend aus dem Wirkstoff Praziquantel (50 % [m/m]) mit einer Partikelgröße d(0,9) = 25 µm, siehe Fig. 1) und den Hilfsstoffen Compritol® 888 ATO (49 % [m/m]), eine Fettgrundlage mit dem Hauptbestandteil Glyceroldibehenat (es enthält auch den Mono- und Triester, sowie kleinere Mengen an Estern mit C₁₆-C₂₀-Fettsäuren), und Aerosil® 200 (1 % [m/m]), ein pyrogenes kolloidales Siliciumdioxid (auch als hochdisperses Siliciumdioxid bezeichnet), dessen Einsatz zur Verbesserung der Fließfähigkeit der Pulvermasse beiträgt, wurde vor der Extrusion in einem Labormischer bei Raumtemperatur gemischt (15 min, 40 U/min) und die Pulvermischung in den gravimetrischen Dosierer des Extruders überfuhrt.

Für die Schmelzextrusion wurde ein gleichläufiger Zweischneckenextruder mit einem Rondenwerkzeug und stumpfen Schneckenaufsätzen verwendet.

Im Laufe der Extrusion dieser Rezeptur verstopften zahlreiche Düsenlöcher (die Düsenplatte hat insgesamt 67 Düsenlöcher mit einem Durchmesser von jeweils 0,3 mm), dadurch stieg der Druck bei gleich bleibender Drehzahl und Dosierrate kontinuierlich an (Fig. 2). Durch den Druckaufbau hinter der Düsenplatte während des Prozesses mit ungemahlenem Praziquantel erhöhte sich die Austrittsgeschwindigkeit der Extrudate aus den verbleibenden freien Düsenlöchern sehr stark.

### II. Beispiel: Extrudat mit fein gemahlenem Praziquantel

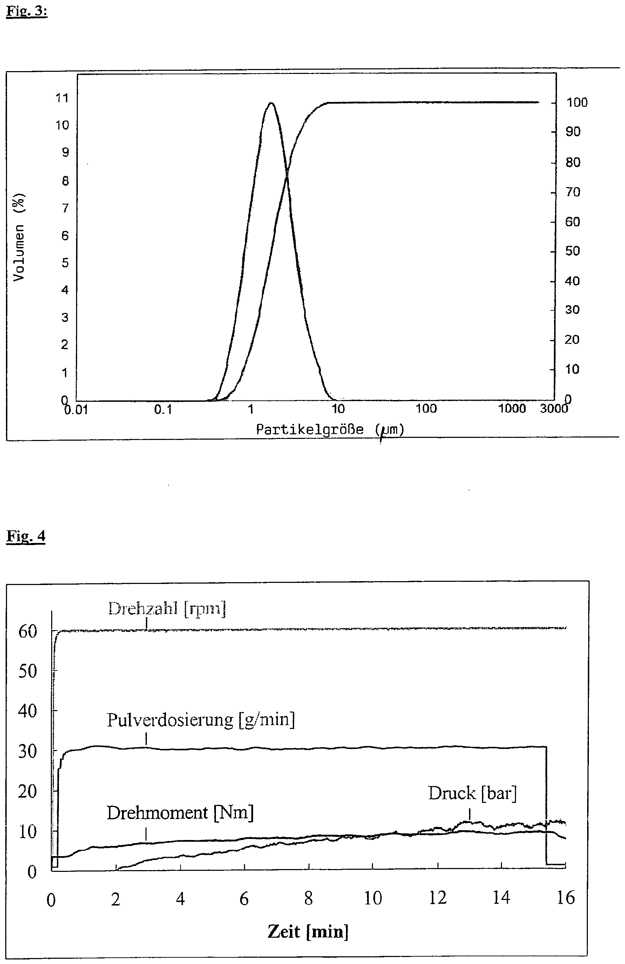

Der Versuch aus dem Vergleichsbeispiel wurde unter Verwendung von Praziquantel wiederholt, das zweimal in einer Luftstrahlmühle gemahlen worden war (d(0,5) = 1,7 µm, d(0,9) = 3,6 µm, siehe Fig. 3).

Der Extrusionsprozess mit gemahlenem Praziquantel war bei einer Arzneistoffbeladung von 50 % und einem Düsendurchmesser von 0,3 mm gleichmäßig und reproduzierbar mit konstantem Druck. Aus dem Prozessverlauf in Fig. 4 ist ersichtlich, dass der Druck sich nach etwa 12 Minuten auf ein konstantes Niveau eingependelt hat und mit etwa 10 bar deutlich niedriger lag als in dem Prozess mit ungemahlenem Praziquantel (Fig. 2), wo der Druck nach 15 Minuten Prozesszeit auf knapp 40 bar angestiegen war. Es wurde durch alle Düsenlöcher mit gleichmäßiger Geschwindigkeit extrudiert.

Beim Vergleich der Extrudate aus dem Vergleichsbeispiel I und diesem Beispiel zeigen sich Unterschiede: Durch die starke Reibung wurde vermehrt Lipid an die Oberfläche der Extrudate gepresst, was unter dem Rasterelektronenmikroskop sehr gut zu sehen ist, die Oberfläche des Extrudates ist glatt und eben. Im Gegensatz dazu ist die Oberfläche des Extrudates mit gemahlenem Praziquantel uneben, die Praziquantelpartikel befinden sich direkt unter der Oberfläche und ihre Form zeichnet sich durch die dünne Lipidschicht ab.

### III. Vergleichsbeispiel: Extrudat mit ungemahlenem Mesalazin

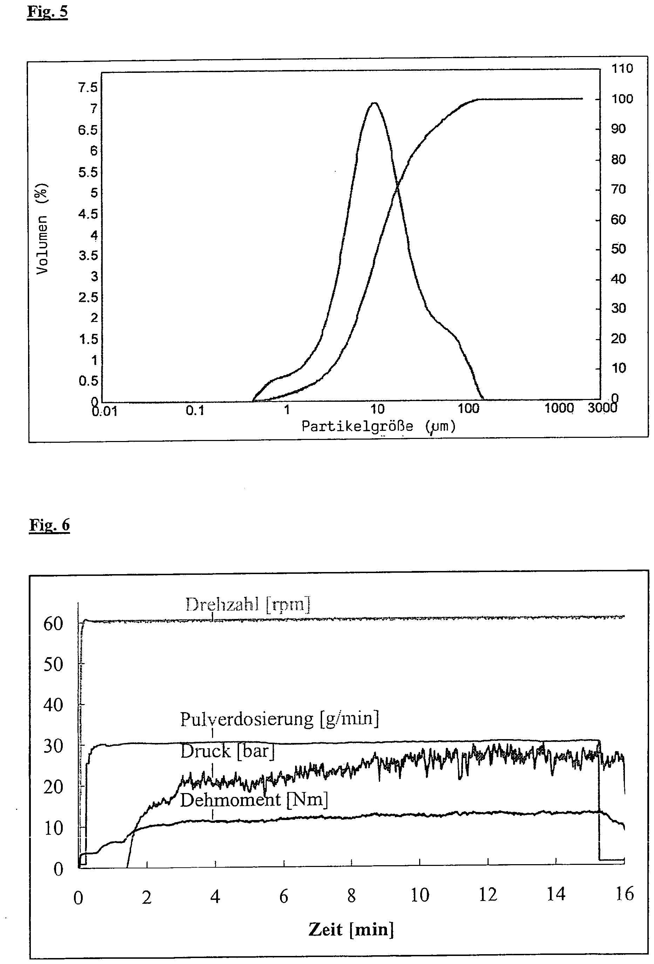

Es wurden Extrudate mit ungemahlenem Mesalazin (d(0,5) = 10,7 µm, d(0,9) = 44,0 µm, vgl. Fig. 5) analog zu Vergleichsbeispiel I hergestellt. Eingesetzt wurde ein Pulvergemisch aus 50 % [m/m] ungemahlenem Mesalazin / 49 % [m/m] Compritol® / 1 % [m/m] hochdisperses Siliciumdioxid (Aerosil®), extrudiert wurde durch eine Düsenplatte mit 0,3 mm Durchmesser. Es traten ähnliche Probleme im Herstellungsprozess auf wie bei Vergleichsbeispiel I. Aus dem Prozessverlauf (Fig. 6) ist ersichtlich, dass der Druck sich nach 10 Minuten bei etwa 25 bar eingependelt hat, aber noch recht stark schwankt. Auch bei diesem Versuch wurde eine teilweise Verstopfung der Düsenlöcher beobachtet, die allerdings nicht so schnell und nicht so großem Ausmaß auftrat wie bei Praziquantel.

### IV. Beispiel: Extrudat mit gemahlenem Mesalazin

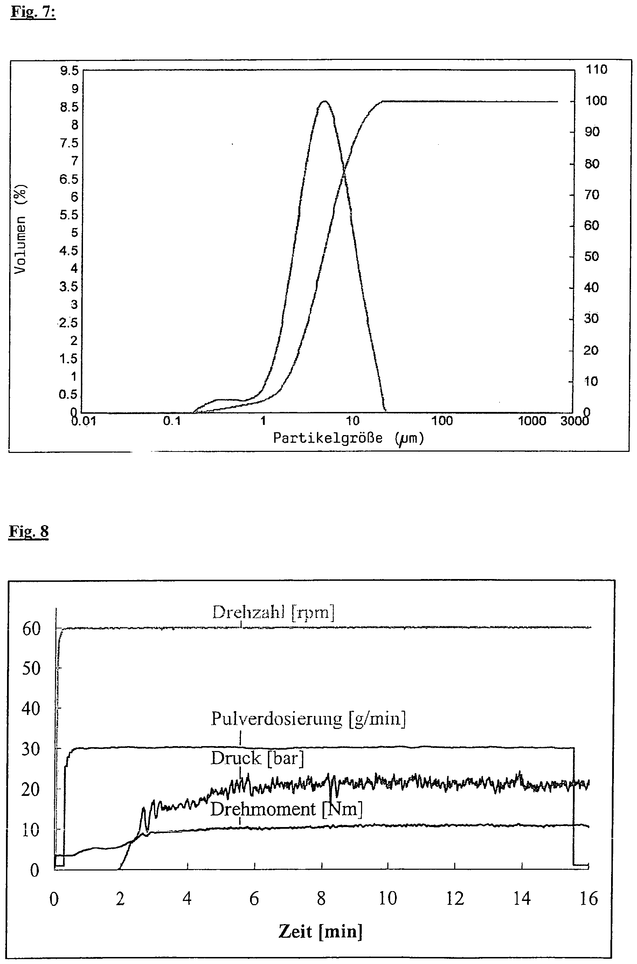

Durch zweimalige Mahlung in einer Luftstrahlmühle wurde Mesalazin mit einer kleineren Partikelgröße erhalten: d(0,5) = 4,9 µm, d(0,9) = 11,9 µm, siehe Fig. 7. Die Partikel zeigten nach der Mahlung eine deutlich veränderte Form.

Durch Einsatz dieses gemahlenen Mesalazin in dem Extrusionsprozess analog Vergleichsbeispiel III, konnte der Prozess entscheidend verbessert werden. In Fig. 8 ist zu sehen, dass der Druck sich schon nach 5 Minuten auf ein konstantes Niveau von 20 bar eingependelt hat, es wurde durch alle Düsenlöcher mit gleichmäßiger Geschwindigkeit extrudiert. Durch Einsatz von gemahlenem Mesalazin konnte also die Verstopfung der Düsenlöcher verhindert und der Extrusionsprozess damit verbessert werden.

Beim Vergleich der Extrudate zeigt sich, dass die nadelförmigen Mesalazin Kristalle aus dem Vergleichsbeispiel während der Extrusion weitgehend zerstört wurden, während das gemahlene Mesalazin Pulver aus diesem Beispiel nach der Extrusion noch seine ursprüngliche Partikelform und -größe aufwies, was z. B unter dem Lichtmikroskop mit Hilfe eines beheizbaren Objektträgers gut zu sehen ist. Die nadelförmigen Praziquantel Kristalle hingegen wurden während der Extrusion nicht zerstört und lagen auch im Extrudat noch in ihrer ursprünglichen Form und Größe vor.

Der Fachmann hätte aufgrund der oben beschriebenen Ergebnisse erwartet, dass die großen nadelförmigen Mesalazin Kristalle bei der Extrusion stärkere Probleme bereiten als die kleineren Praziquantelnadeln. Doch die Verstopfung der Düsenlöcher und der dadurch bedingte schnelle Druckaufbau traten mit Mesalazin aus dem Grund nicht so stark auf wie mit Praziquantel, weil die großen Mesalazin Nadeln schon während des Prozesses größtenteils durch die Scherwirkung der Extruderschnecken zerkleinert wurden.

### V. Vergleichsbeispiel: Extrudat ohne Antistatikum

Es wurden Extrudate mit gemahlenem Praziquantel analog zu Beispiel II hergestellt. Eingesetzt wurde ein Pulvergemisch aus 50 % [m/m] gemahlenem Praziquantel / 49 % [m/m] Dynasan 116® / 1 % [m/m] hochdisperses Siliciumdioxid (Aerosil®), bei Dynasan 116® handelt es sich um eine Fettgrundlage, die zu 98 % aus Glyceroltripalmitat besteht. Extrudiert wurde durch eine Düsenplatte mit 0,3 mm Durchmesser.

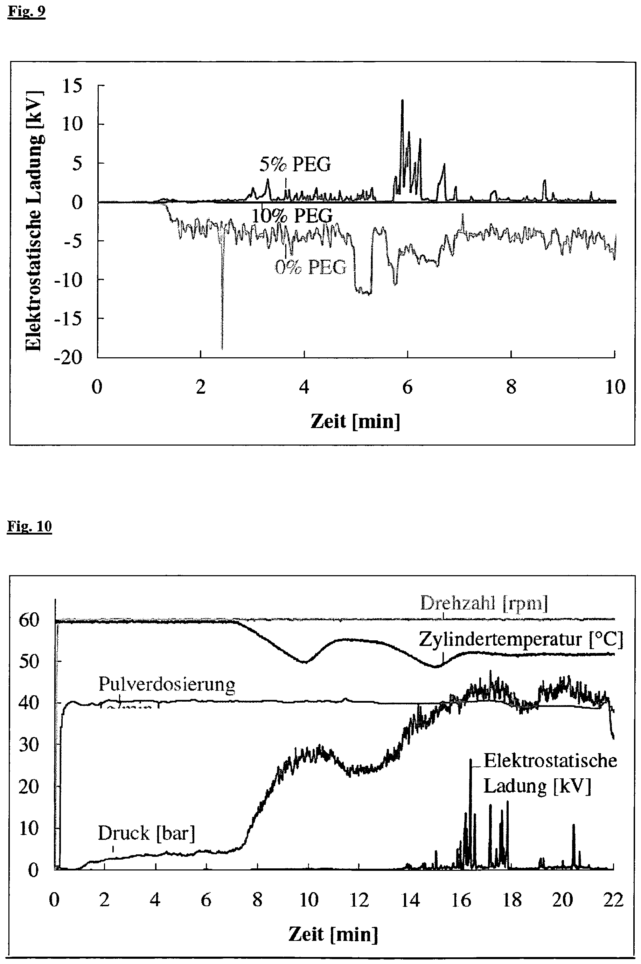

Im Laufe der Extrusion dieser Rezeptur entstanden immer stärkere elektrische Ladungen an den Extrudaten durch Reibung der lipophilen Masse an den Düseninnenflächen, so dass schon nach wenigen Minuten das Extrudat vom Extruder selbst stark elektrostatisch angezogen wurde und am Düsenkopf haften blieb. Bedingt durch diese Aufladung verstopften auch einige Düsenlöcher und der Druck hinter der Düsenplatte stieg an, obwohl gemahlenes Praziquantel verwendet wurde. Die auftretenden Ladungen konnten mit Hilfe des elektrostatischen Sensors IZD 10-510 der Firma SMC, der während des Prozesses direkt vor der Düsenplatte angebracht war, kontinuierlich aufgezeichnet werden. Während der Extrusion dieses Vergleichsbeispiels erreichte die elektrostatische Ladung bereits nach knapp 2 Minuten einen Wert von -5 kV (Fig. 9).

### VI. Beispiel: Extrudat mit Antistatikum

Es wurden Extrudate unter Zusatz von 5 und 10 % PEG 1500 als Antistatikum analog zu Vergleichsbeispiel V hergestellt. Eingesetzt wurden Pulvergemische aus 50 % [m/m] gemahlenem Praziquantel / 44 % [m/m] Dynasan 116® / 5 % [m/m] PEG 1500 / 1 % [m/m] Aerosil®, bzw. aus 50 % [m/m] gemahlenem Praziquantel / 39 % [m/m] Dynasan 116® / 10 % [m/m] PEG 1500 / 1 % [m/m] hochdisperses Siliciumdioxid (Aerosil®). Die Zylindertemperatur lag bei 60°C, was ein Schmelzen des PEG 1500 während des Extrusionsprozesses gewährleistete.

Im Laufe der Extrusion mit 5 % PEG verstopften erneut einige Düsenlöcher, nach 3 Minuten Prozesszeit wurden elektrostatische Ladungen von 1-2 kV vor der Düsenplatte gemessen. Der Extrusionsprozess mit 10 % PEG dagegen verlief gleichmäßig und reproduzierbar, ohne verstopfte Düsenlöcher und ohne messbare elektrostatische Aufladung (Fig. 9).

Durch Zusatz von 10 % PEG als Antistatikum konnten elektrostatische Aufladungen des Extrudates mit hochreinen Glyceroltriestern verhindert und der Prozess damit deutlich verbessert werden.

### VII. Vergleichsbeispiel: Extrudat mit ungeschmolzenem Antistatikum

Es wurden Extrudate unter Zusatz von 10 % PEG 6000 als Antistatikum analog zu Beispiel VI hergestellt. Eingesetzt wurde ein Pulvergemisch aus 50 % [m/m] gemahlenem Praziquantel / 39 % [m/m] Dynasan 116® 10 % [m/m] PEG 6000 / 1 % [m/m] hochdisperses Siliciumdioxid (Aerosil®). Die Zylindertemperatur lag zunächst bei 60°C und wurde nach 8 Minuten zunächst auf 55°C und schließlich auf 52°C abgekühlt. Der Schmelzbereich von PEG 6000 liegt bei 55-60°C, so dass das PEG nach Abkühlen der Zylindertemperatur nicht mehr geschmolzen vorlag.

Der Prozess verlief zunächst, analog zu Beispiel VI mit 10 % PEG 1500, gleichmäßig und frei von elektrostatischen Ladungen, alle Düsenlöcher waren frei (Fig. 10). Nach Absenken der Temperatur luden sich die Extrudate elektrostatisch auf, einige Düsenlöcher verstopften und dadurch bedingt stieg der Druck hinter der Düsenplatte an.

Es konnte gezeigt werden, dass das PEG während des Extrusionsprozesses geschmolzen vorliegen sollte, um seine antistatische Wirkung zu entfalten.

### VIII. Vergleichsbeispiel: Extrudat mit ungemahlenem Coffein

Es wurden Extrudate mit ungemahlenem Coffein analog zu Vergleichsbeispiel I hergestellt. Eingesetzt wurde ein Pulvergemisch aus 50 % [m/m] ungemahlenem Coffein (Partikelgröße d(0,9) = 1170 µm) / 49 % [m/m] Compritol® / 1 % [m/m] hochdisperses Siliciumdioxid (Aerosil®); extrudiert wurde durch eine Düsenplatte mit 0,3 mm Durchmesser. Es traten ähnliche Probleme im Herstellungsprozess auf wie bei Vergleichsbeispiel I. Bereits nach 8 Minuten Extrusion stieg der Druck durch eine vollständige Verstopfung der Düsenlöcher so stark an, dass der Prozess abgebrochen werden musste.

### IX. Beispiel: Extrudat mit gemahlenem Coffein

Durch zweimalige Mahlung in einer Luftstrahlmühle wurde Coffein mit einer kleineren Partikelgröße erhalten.

Durch Einsatz dieses gemahlenen Coffeins in dem Extrusionsprozess analog Beispiel II konnte der Prozess entscheidend verbessert werden. Bei der Extrusion pendelte sich der Druck schon nach 3 Minuten auf ein konstantes Niveau von 5 bar ein, es wurde durch alle Düsenlöcher mit gleichmäßiger Geschwindigkeit extrudiert. Durch Einsatz von gemahlenem Coffein konnte also die Verstopfung der Düsenlöcher verhindert und der Extrusionsprozess deutlich verbessert werden.

Analog zu den vorstehende Beispielen wurden folgende Extrudate hergestellt:

### X. Beispiel

| | |
|---|---|
| 70 % [m/m] | Praziquantel (gemahlen, d(0,9) = 5,7 µm) |
| 17 % [m/m] | Glyceroltristearat (Dynasan® 118) |
| 12 % [m/m] | PEG 6000 |
| 1 % [m/m] | hochdisperses Siliciumdioxid (Aerosil®) |

Düsendurchmesser: 0,3 mm (100 % freie Düsenlöcher bei der Extrusion), Schneckengeschwindigkeit: 60 UpM, Extrusionstemperatur: 67 °C.

### XI. Beispiel

| | |
|---|---|
| 50 % [m/m] | Praziquantel (gemahlen, d(0,9) = 5,7 µm) |
| 29 % [m/m] | Glyceroltristearat (Dynasan® 118) |
| 20 % [m/m] | PEG 6000 |
| 1 % [m/m] | hochdisperses Siliciumdioxid (Aerosil®) |

Düsendurchmesser: 0,2 mm (180 Düsenlöcher, 100 % freie Düsenlöcher bei der Extrusion), Schneckengeschwindigkeit: 60 UpM, Extrusionstemperatur: 67 °C.

### XII. Beispiel

| | |
|---|---|
| 50 % [m/m] | Praziquantel (gemahlen, d(0,9) = 5,7 µm) |
| 29 % [m/m] | Glyceroltristearat (Dynasan® 118) |
| 20 % [m/m] | PEG 6000 |
| 1 % [m/m] | hochdisperses Siliciumdioxid (Aerosil®) |

Düsendurchmesser: 0,3 mm (100 % freie Düsenlöcher bei der Extrusion), Schneckengeschwindigkeit: 60 UpM, Extrusionstemperatur: 67 °C.

### XIII. Beispiel

| | |
|---|---|
| 50 % [m/m] | Praziquantel (gemahlen, d(0,9) = 5,7 µm) |
| 49 % [m/m] | Glyceroltristearat (Dynasan® 118) |
| 1 % [m/m] | hochdisperses Siliciumdioxid (Aerosil®) |

Düsendurchmesser: 0,3 mm (3 % freie Düsenlöcher bei der Extrusion), Schneckengeschwindigkeit: 60 UpM, Extrusionstemperatur: 67 °C.

### Biologisches Beispiel: Akzeptanztest an Katzen

Vierzig Katzen wurden in 4 Gruppen zu je 10 Tieren aufgeteilt. Je ein Extrudat der Beispiele X bis XIII wurde mit jeweils einer Gruppe von 10 Katzen auf folgende Weise getestet:
Die Menge an Extrudat, die einer Dosierung von 5mg Praziquantel pro kg Körpergewicht entspricht, wurde als Topdressing auf Katzen-Trockenfutter gegeben und den Katzen zu den normalen Fütterungszeiten angeboten. Alle Katzen fraßen das gesamte Futter. Dies wurde als Beleg für 100 % Akezptanz gewertet.

Nach einer einwöchigen Pause wurde die Untersuchung wiederholt, wobei jedoch Dosenfutter (feucht) für Katzen verwendet wurde. Es wurde das gleiche Ergebnis wie bei dem Test mit Trockenfutter beobachtet und als 100 % Akzeptanz bewertet.

### Figuren

- Fig. 1:: Partikelgrößenverteilung von ungemahlenem Praziquantel, d(0,5) = 6,2 µm, d(0,9) = 25,1 µm, gemessen per Laserdiffraktometrie nach Feuchtdispergierung (Mastersizer 2000 Ver. 5.54, Malvern Instruments, Malvern UK).
- Fig. 2:: Prozessverlauf der Extrusion mit 50 % ungemahlenem Praziquantel / 49 % Compritol® / 1 % hochdisperses Siliciumdioxid (Aerosil®), Düsenplatte 0,3 mm Durchmesser.
- Fig. 3:: Partikelgrößenverteilung von zweifach gemahlenem Praziquantel, d(0,5) = 1,7 µm, d(0,9) = 3,6 µm, gemessen per Laserdiffraktometrie nach Feuchtdispergierung (Mastersizer 2000 Ver. 5.54, Malvern Instruments, Malvern UK).
- Fig. 4:: Prozessverlauf der Extrusion mit 50 % gemahlenem Praziquantel / 49 % Compritol® / 1 % hochdisperses Siliciumdioxid (Aerosil®), Düsenplatte 0,3 mm Durchmesser.
- Fig. 5:: Partikelgrößenverteilung von ungemahlenem Mesalazin, d(0,5) = 10,7 µm, d(0,9) = 44,0 µm, gemessen per Laserdiffraktometrie nach Feuchtdispergierung (Mastersizer 2000 Ver. 5.54, Malvern Instruments, Malvern UK).
- Fig. 6:: Prozessverlauf der Extrusion mit 50 % ungemahlenem Mesalazin / 49 % Compritol® / 1 % hochdisperses Siliciumdioxid (Aerosil®), Düsenplatte 0,3 mm Durchmesser.
- Fig. 7:: Partikelgrößenverteilung von zweifach gemahlenem Mesalazin, d(0,5) = 4,9 µm, d(0,9) = 11,9 µm, gemessen per Laserdiffraktometrie nach Feuchtdispergierung (Mastersizer 2000 Ver. 5.54, Malvern Instruments, Malvern UK).
- Fig. 8:: Prozessverlauf der Extrusion mit 50 % gemahlenem Mesalazin / 49 % Compritol® / 1 % hochdisperses Siliciumdioxid (Aerosil®), Düsenplatte 0,3 mm Durchmesser.
- Fig. 9:: Elektrostatische Ladung vor der Düsenplatte während des Extrusionsprozesses mit 0, 5, bzw. 10 % PEG 1500 Anteil.
- Fig. 10:: Prozessverlauf der Extrusion mit 50 % gemahlenem Praziquantel / 39 % Dynasan 116® / 10 % PEG 6000 / 1 % hochdisperses Siliciumdioxid (Aerosil®).

## Patentansprüche

1. Extrudate, enthaltend mindestens eine pharmazeutisch wirksame Substanz in Form von Nadeln, **dadurch gekennzeichnet, dass** das Verhältnis von Partikelgröße der nadelförmigen pharmazeutisch wirksamen Substanz zu Strangdurchmesser mindestens 1:20 beträgt,
dass das Extrudat eine Lipidgrundlage als Hilfsstoff enthält und
dass das Extrudat unterhalb der Untergrenze des Schmelzbereichs der darin enthaltenen Grundlage extrudiert wurde.

2. Extrudate gemäß Anspruch 1, mit einem Verhältnis von Partikelgröße der nadelförmigen pharmazeutisch wirksamen Substanz zu Strangdurchmesser von mindestens 1:25.

3. Extrudate gemäß einem der vorstehenden Ansprüche, mit einem Strangdurchmesser von 0,5 mm oder weniger.

4. Extrudat gemäß einem der vorstehenden Ansprüche, enthaltend einen Glycerolester mit C₁₂-C₂₄-Fettsäuren als Lipidgrundlage.

5. Extrudat gemäß einem der vorstehenden Ansprüche, enthaltend einen Glyceroldiester als Lipidgrundlage.

6. Extrudat gemäß Anspruch 5, enthaltend Glyceroldibehenat als Lipidgrundlage.

7. Extrudat gemäß einem der Ansprüche 1 bis 4, enthaltend einen Glyceroltriester als Lipidgrundlage.

8. Extrudat gemäß Anspruch 7, enthaltend Glyceroltrimyristat, Glyceroltripalmitat oder Glyceroltristearat als Lipidgrundlage.

9. Extrudat gemäß Anspruch 8, enthaltend Glyceroltristearat als Lipidgrundlage.

10. Extrudat, gemäß einem der vorstehenden Ansprüche, enthaltend ein Antistatikum, insbesondere ein Polyethylenglykol.

11. Verwendung von Extrudaten gemäß einem der vorstehenden Ansprüche zur Herstellung von Arzneimitteln.

12. Arzneimittel, enthaltend ein Extrudat gemäß einem der Ansprüche 1 bis 10 sowie einen oder mehrere pharmazeutisch annehmbare Hilfs- und/oder Zusatzstoffe.

## Claims

1. Extrudates containing at least one pharmaceutically active substance in the form of needles, **characterized in that** the ratio of the particle size of the needle-shaped pharmaceutically active substance to the strand diameter is at least 1:20,
**in that** the extrudate contains a lipid base as an additive and
**in that** the extrudate was extruded below the lower limit of the melting range of the base contained therein.

2. Extrudates according to Claim 1, **characterized in that** the ratio of the particle size of the needle-shaped pharmaceutically active substance to the strand diameter is at least 1:25.

3. Extrudates according to one of the previous claims, **characterized in that** the strand diameter is 0.5 mm or less.

4. Extrudate according to one of the previous claims, **characterized in that** it contains a glycerol ester with C₁₂-C₂₄ fatty acids as the lipid base.

5. Extrudate according to one of the previous claims, **characterized in that** it contains a glycerol diester as the lipid base.

6. Extrudate according to Claim 5, **characterized in that** it contains glycerol dibehenate as the lipid base.

7. Extrudate according to one of Claims 1 to 4, **characterized in that** it contains a glycerol triester as the lipid base.

8. Extrudate according to Claim 7, **characterized in that** it contains glycerol trimyristate, glycerol tripalmitate or glycerol tristearate as the lipid base.

9. Extrudate according to Claim 8, **characterized in that** it contains glycerol tristearate as the lipid base.

10. Extrudate according to one of the previous claims, **characterized in that** it contains an antistatic agent, in particular a polyethylene glycol.

11. Use of extrudates according to one of the previous claims for the production of medicaments.

12. Medicament **characterized in that** it contains an extrudate according to one of Claims 1 to 10 and one or more pharmaceutically acceptable auxiliary substances and/or additives.

## Revendications

1. Produits extrudés, contenant au moins une substance pharmaceutiquement active sous forme d'aiguilles, **caractérisés en ce que** le rapport de la grosseur de particule de la substance pharmaceutiquement active sous forme d'aiguilles au diamètre du brin est d'au moins 1:20, **en ce que** le produit extrudé contient une base lipidique comme adjuvant et **en ce que** le produit extrudé a été extrudé sous la limite inférieure de la plage de fusion de la base qui y est contenue.

2. Produits extrudés selon la revendication 1, présentant un rapport de la grosseur de particule de la substance pharmaceutiquement active sous forme d'aiguilles au diamètre du brin d'au moins 1:25.

3. Produits extrudés selon l'une quelconque des revendications précédentes, présentant un diamètre de brin de 0,5 mm ou moins.

4. Produit extrudé selon l'une quelconque des revendications précédentes, contenant un ester de glycérol avec des acides gras en C₁₂-C₂₄ comme base lipidique.

5. Produit extrudé selon l'une quelconque des revendications précédentes, contenant un diester de glycérol comme base lipidique.

6. Produit extrudé selon la revendication 5, contenant du dibéhénate de glycérol comme base lipidique.

7. Produit extrudé selon l'une quelconque des revendications 1 à 4, contenant un triester de glycérol comme base lipidique.

8. Produit extrudé selon la revendication 7, contenant du trimyristate de glycérol, du tripalmitate de glycérol ou tristéarate de glycérol comme base lipidique.

9. Produit extrudé selon la revendication 8, contenant du tristéarate de glycérol comme base lipidique.

10. Produit extrudé selon l'une quelconque des revendications précédentes, contenant un antistatique, en particulier un polyéthylèneglycol.

11. Utilisation de produits extrudés selon l'une quelconque des revendications précédentes pour la préparation de médicaments.

12. Médicament contenant un produit extrudé selon l'une quelconque des revendications 1 à 10 ainsi qu'un ou plusieurs adjuvants et/ou additifs pharmaceutiquement acceptables.
